# EUROPEAN PATENT SPECIFICATION

(11) **EP 2 528 871 B1**
(45) Date of publication and mention of the grant of the patent: **07.12.2016**
(21) Application number: 11701359.9
(22) Date of filing: 25.01.2011
(51) Int. Cl.: C02F 11/04, A01C 3/00, C05F 3/00, C02F 101/10, C02F 3/28, C02F 1/44, C02F 103/20

(54) **METHOD FOR DEPHOSPHORIZING MANURE AND/OR BIOMASS**
VERFAHREN ZUR ENTPHOSPHORIERUNG VON DUNG UND/ODER BIOMASSE
PROCÉDÉ POUR DÉPHOSPHORER UN FUMIER ET/OU UNE BIOMASSE

(30) Priority: 25.01.2010 NL 2004145
(43) Date of publication of application: 05.12.2012
(73) Proprietor: Ceres Milieu Holding BV, 7609 PG Almelo (NL)
(72) Inventor: KRUIDHOF, Hendrik, 7609 PG Almelo (NL); KRUIDHOF, Henriette Marjolein, 6706 DV Wageningen (NL)
(74) Representative: van Kooij, Adriaan
(86) International application number: PCT/EP2011/000305
(87) International publication number: WO 2011/089019

(56) References cited:
- EP-A1- 0 134 766
- WO-A1-2009/072887
- DE-A1- 4 000 834
- DE-A1- 19 937 876
- ES-A1- 2 292 277
- Dauber S.: "Anaerobtechnik. Handbuch der anaeroben Behandlung von Abwasser und Schlamm", 1993, Springer, Berlin, XP002599329, ISBN: 3-540-56410-1 figure 6.4 page 340 - page 341
- YANAGI C ET AL: "Treatment of wheat starch waste water by a membrane combined two phase methane fermentation system", DESALINATION, ELSEVIER, AMSTERDAM, NL LNKD- DOI:10.1016/0011-9164(94)00141-3, vol. 98, no. 1, 1 September 1994 (1994-09-01), pages 161-170, XP004019242, ISSN: 0011-9164
- EJ Nyns: "Methane", Ullmann's Encyclopedia of Industrial Chemistry , 15 June 2000 (2000-06-15), pages 1-11, XP002632095, DOI: 10.1002/14356007.a16_453 Retrieved from the Internet: URL:http://onlinelibrary.wiley.com/doi/10. 1002/14356007.a16_453/pdf [retrieved on 2011-04-08]
- JEFF TATE: 'An introduction to spiral wound EDI', [Online] 01 January 2003, XP055275074 Retrieved from the Internet: <URL:http://www.agapewater.com/wp-content/u ploads/2014/08/watertechnologyjan03.pdf> [retrieved on 2016-05-24]

## Description

The present invention relates to a method for processing manure and/or biomass, which can be used for the production of biogas and results in the provision of a solid fraction having a strongly reduced phosphorus content and in the provision of a liquid fraction with a strongly increased phosphorus content compared to a solid fraction.

In many West-European countries, intensive livestock rearing has led to problems of manure surplus because the cycle of fertilizing farmlands, rearing livestock or growing crops to feed livestock and the resulting production of manure from this livestock has become unbalanced. In many countries current legislation prevents the return of large amounts of manure to farmland as fertilizer because of the occurrence of adverse environmental effects that are due to the accumulation of nutrients in natural ecosystems. Thus, in order to prevent the accumulation of unacceptable amounts of manure, it needs to be processed into other useful products which can find their use in alternative fields or as an alternative product in the same field.

One of these processes involves the fermentation, in the art also known as anaerobic digestion, of manure. Complete fermentation of manure results in the production of mainly methane and carbon dioxide, and a not fermented fraction, which in the art is termed digestate. This latter fraction is normally used as a fertilizer in the same way as manure.

Fermentation of manure, or biomass, is a rather complex process mediated by several coexisting populations of different microorganism species, mainly bacteria and archaea, which together are able to convert organic material, with biogas as a final product. Biogas is an energy source and can be used to replace fossil fuels. It consists mainly of methane and carbon dioxide, and can contain traces of other gases such as hydrogen and hydrogen sulphide. This anaerobic process can be divided into the following four sub-processes or phases: Hydrolysis, acidogenesis, acetogenesis and methanogenesis. During these consecutively proceeding phases, larger biodegradable molecules such as carbohydrate polymers, proteins and fats are converted into smaller compounds, which eventually leads to the production of biogas. Furthermore, the not fermented digestate is obtained as a residual fraction.

At present, manure and/or biomass can be processed into digestate by fermenting it in a one-step fermentation process performed in a single reactor, or in a two-step fermentation process performed in at least two separated reactors.

In a one-step fermentation process, the entire process comprising the four above mentioned consecutive fermentation phases proceed simultaneously in a single reactor. Because the various microorganism species that are involved in the distinct fermentation stages have different pH optima, the pH in the reactor is kept at a compromise to allow all four fermentation phases to proceed. Generally, the pH is held between 6.5 and 8.5, preferably 7.2 and 8.2. However, because the methanogenic microorganisms are quite sensitive to pH fluctuations, when biogas production is the main objective, the degree of acidity of the fermenting organic material in the reactor is preferably kept in a rather narrow window of around pH 7.5 to obtain the best results. A large drop in pH is not desirable since this adversely affects the biogas production rate.

WO 2009/004027 discloses a method of recovering phosphate from fermented biomass such as animal manure, waste slurries or organically loaded waste water. The document ES2292277 discloses another known method for recovering phosphate from fermented biomass.

In a two-step fermentation process, the manure and/or biomass is acidified in a first reactor where the hydrolysis, acidogenesis and partially also acetogenesis takes place. Next, the acidified manure and/or biomass is fed into a separate, second reactor where the fermentation process continues, meaning further acetogenesis and the methanogenesis leading to biogas production. One advantage of the two-step fermentation process is that biogas production can be higher than in the one-step configuration due to improved decomposition of organic material in the acidifying reactor.

Irrespective of the choice of fermentation via the one-step or two-step process, after completion of both alternative processes, digestate remains as a residual fraction. This digestate can subsequently be divided in two fractions: a solid, mainly organic fraction and a liquid, mainly inorganic fraction. When produced from manure, this liquid fraction typically has a low phosphorus content and a high nitrogen and potassium content of about 10% P, 90% N and 90% K. Consequently, the solid fraction has a high phosphorus content and low nitrogen and potassium content. Disposal of solid organic fraction having high phosphorus content may present a problem for the relevant agricultural industries because in many jurisdictions only a limited amount of nutrients, in particular phosphorus-comprising nutrients, may be added to farmland. Consequently, not all of the solid fraction may be used as fertilizer due to the prevailing legislation.

One of the challenges that the intensive livestock industry has been facing for the last 25 years is to reduce the production of manure to prevent the occurrence of problems associated with manure surplus, or to reduce the surplus manure or to process the manure to lower the nutrient content, or to produce feedstock or (raw) starter material for other industries. So far though, an economically viable method for processing of manure into marketable and commercially interesting end-products has not been developed yet.

Consequently, there remains a need for the development of methods that can aid in solving issues associated with surplus manure or similar issues associated with accumulation of other types of biomass.

It is therefore an object of the present invention to provide a solution to the above-mentioned problems. This object, among others, is met by the method according to the appended claims.

Disclosed is a method for processing of manure, biomass or a combination thereof, comprising the following steps:
a) fermentation of manure in a first reactor in an acidic environment,
b) separation of the fermented manure into a liquid and a solid fraction,
c) fermentation of the liquid fraction in a second reactor in a basic environment, preferably neutral to basic environment.

Additionally or alternatively, disclosed is a method for processing of manure and/or biomass comprising the steps of:
a) fermenting the manure and/or biomass in a first reactor to obtain acidified manure and/or biomass having a pH of below 6, preferably below pH 5.5, more preferably below 5,
b) separating the acidified manure and/or biomass into a liquid fraction and a solid fraction, and
c) adding the liquid fraction to a second reactor, which second reactor comprises fermenting liquid fraction having a pH of between 6.5 and 8.5, preferably 7.0 to 8.0, and fermenting the thus obtained mixture in the second reactor.

An advantage of this process is that the acidification of manure in the first reactor (herein also functionally referred to as "acidifying reactor") results in the formation of complexes comprising of phosphates and naturally-formed fatty acids, in particular volatile fatty acids. Naturally-formed means that these fatty acids are produced as a consequence of the degradation of large organic polymers that decompose during the hydrolytic and acidogenic phases of anaerobic fermentation of the organic material comprised by the first reactor.

In previously known methods of manure and/or biomass fermentation, phosphorus comprised by the starting material mainly ends up in the solid fraction after the separation of digestate into solid and liquid fractions. In the method of the present invention, the distribution of phosphorus shifts from the solid fraction towards the liquid fraction because during fermentation the phosphorus is liberated from the organic fraction after which it becomes solubilized as phosphate at the prevailing low pH values in the acidifying reactor. The main relevance thereof is that phosphate can be readily recovered from the liquid fraction, which is not the case for phosphorus-comprising compounds comprised by solid fractions. Thus, the present invention allows a more efficient recovery of phosphorus, in particular in the form of phosphates, from manure and/or biomass.

The incubation period of material comprised by the first reactor may vary, but since phosphates will become dissolved at low pH values (i.e. below pH 6 or 5), it can readily be established by determining the pH value when the manure has sufficiently fermented such that the acidified manure is to be fed into a separating means to separate the manure in a solid and liquid fraction. Monitoring of the pH during fermentation of the contents of the acidifying reactor thus enables the skilled person to determine the moment when the manure is to be separated in a liquid and a solid fraction. At these low pH values, the phosphorus encompassed by the contents of the first reactor is solubilized as phosphate, which thus allows the provision of a phosphate-poor solid fraction and phosphate-enriched liquid fraction. Consequently, the outflow of the first reactor is pH-regulated allowing for a controlled outflow from the first reactor, such that material having a pH below pH 6.0, preferably 5.5, or even below pH 5.0, is released from the first reactor for supply to the separator.

Obviously, it is also possible to measure the amounts of dissolved phosphates in the reactor to select the moment of separating the manure in step b). Measuring the pH of the material comprised by the first reactor also allows the skilled person to monitor the acidifying process. For example, more biomass, or biomass with a high content of fermentable organic matter (e.g. starch, maize kernels, potatoes et cetera), can be added to the first reactor resulting in a more rapid decrease in pH such that the preferred pH value is achieved in a shorter time period.

After the separation in step b), most of the phosphorus, preferably more than 90% thereof, is present in the liquid fraction whereas the solid fraction contains the remaining proportion. Similar distributions over the solid and liquid fraction also apply to dissolved potassium and nitrogen comprising minerals. Separation of the acidified manure thus leads to a mineral-poor solid fraction and mineral-enriched liquid fraction. The liquid fraction contains preferably between 50-100% of the total phosphorus, more preferably between 70-100% and most preferably between 90-100%. Since natural phosphate resources are becoming increasingly limited, it is of interest to be able to recover phosphorus from manure and/or biomass which has become possible by the method of the present invention.

Another advantage of the present invention is that phosphate-poor solid fractions can be used as fertilizer, such as after composting thereof. The phosphorus and nitrogen content of the thus obtained solid fraction is sufficiently low that more solid fraction can be used as compost or fertilizer without exceeding the lawfully regulated allowable amounts wherein P and/or N can be added. Furthermore, it still contains most of the organic matter present in the manure and/or biomass, which is highly desirable for soil improvement. The solid fraction can be dried and pressed into pellets or granules.

The solid fraction obtained by step b) is washed with water and separated again in a solid and liquid fraction. This washing can be performed with a washer/separator. This step allows the mineral and fatty acid content of the solid fraction to be further lowered such that a non-smelling, mineral-poor and fatty acid-poor solid fraction is obtained.

Furthermore, since phosphorus-poor solid fractions can be used as fertilizer and as fermentation installations producing such solid fractions would mostly be located in rural areas, such solid fractions can be disposed of in the vicinity of the fermentation installation. This implies that the logistics surrounding the transport of solid fractions become less complicated and less costly. Moreover, phosphate can be relatively easily separated in a concentrated form from the liquid fraction, whereas separating phosphate from the solid fraction has so far not been possible.

Another advantage of the present invention is that the use of complexing agents intended to prevent the formation of indissoluble phosphate-comprising complexes prior to separation of manure in solid and liquid fractions is not needed.

For the purpose of the present invention, the term "fermentation" is interchangeable with the term "anaerobic digestion", which both mean the break-down of biodegradable material in the absence of gaseous oxygen.

Besides providing the option of fermenting only manure, the present invention also allows fermenting a mixture of manure and other types of biomass, which process is referred to as co-fermentation.

The term "manure" herein is meant in its arts recognized meaning of excreta from farm animals. Where necessary, this term is specified by referring to specific farm animals, such as pigs, cattle, cows, horses and the like.

The term "biomass" as used herein includes waste products, such as organic residues from agricultural, municipal and/or industrial origin comprising a fermentable organic fraction. These residues include plant material and material of animal origin, such as dairy products. Plant material specifically includes municipal fruit and vegetable waste, but also agricultural residues, such as material from plants such as maize, sugar beet, cereal plants, soybean, potato, grasses and the like. Depending on local legislation, biomass herein also includes sludges derived from municipal or industrial wastewater treatment, and human excreta e.g. separately collected from vacuum toilets.

In the two-step process of the present invention, the contents of the first reactor are kept at anaerobic conditions allowing the degree of acidity of the fermenting starter material to lower from its starting, neutral pH or slightly basic pH to a more acidic pH during progression through the first stages of fermentation. For phosphorus to become dissolved in the form of phosphates, it is sufficient that the material comprised by the first reactor acidifies to a pH of below 6. However, the pH of the contents of the first reactor may decrease further to pH values of below 5.5 or below 5.

The first reactor can be operated as a plug flow reactor or as a continuously stirred reactor. The outflow from such reactors is pH-regulated such that when the fermenting material near the outlet reached the preferred pH of below 6, 5.5 or 5, this acidified material is transported to the separator means for separation into solid and liquid fractions. Preferably the first reactor is directly connected to the separator means with a suitable conduit. Thus, the acidified manure obtained by step a) is preferably separated in step b) without an intermediate processing step. The inflow into the first reactor is preferably regulated using a level indicator controller (LIC).

It is also possible to stimulate the acidifying reactions by adding inoculum and/or energy-rich fermentable biomass, such starch-comprising biomass, to the first reactor. The addition of such energy-rich fermentable biomass leads to the production of more fatty acids. It is also possible to add inoculum to the second reactor. The inoculum is preferably taken from a reactor wherein reactions take place that correspond to the reactor to which the inoculum is added.

After treatment in the first reactor, the acidified manure, optionally co-fermented with other biomass types as meant herein, is separated into a solid and liquid fraction by separating means using mechanical and/or gravitational forces. The phosphate-enriched, acidified liquid fraction according to the present invention is fed into the second reactor for further fermentation, production of biogas and precipitation of phosphorus-comprising compounds.

Step c) of the present invention, the addition of liquid fraction to the second reactor, is meant herein to take place during the processing of manure and/or biomass using the installation. This means that the second reactor comprises fermenting liquid fraction from a prior added batch of acidic liquid fraction. However, in case the second reactor would be empty, e.g. when the installation is used for the first time or after maintenance or repair requiring the second reactor to be emptied or replaced, liquid fraction is added to the empty reactor. Optionally, a suitable inoculum and/or biomass is/are added to the liquid fraction to allow initiation of the final stages of fermentation to take place and hence the production of biogas.

According to an advantageous embodiment of the present invention, to the first reactor used in step a) is added biomass and/or a solid fraction of separated manure, preferably unfermented manure. Also possible is to add biomass in combination with the liquid fraction obtained by step b) or a solid fraction of separated manure, preferably unfermented manure, in combination with the liquid fraction obtained by step b) or a combination of biomass and the solid fraction of separated manure, preferably unfermented manure, with the liquid fraction obtained by step b). The addition of this material or the mentioned combinations thereof to the first reactor is meant to allow the process to progress after acidified fermented material is conveyed to the separator means used to separate the organic material in step b). It is advantageous to use a mixture of a solid fraction of separated fresh manure and the liquid fraction obtained by step b) since this causes the pH of the fresh solid fraction of added manure to be pre-acidified. This allows the contents of the first reactor to remain in the preferred acidic range after addition of unfermented manure and thus the acidifying reactions in the first reactor to progress in a more efficient manner. Preferably, the dry matter content of this material added to the first reactor lies between 15 to 20 weight percent.

According to another advantageous embodiment of the present invention, the liquid fraction obtained by step b) is filtered prior to addition thereof to the second reactor used in step c). Preferably, this filtering comprises microfiltration and results in the separation of dry matter particles from the liquid containing the organic acids.

The conditions in the second reactor, herein also functionally referred to as the "biogas reactor", are such that the contents thereof are kept at neutral to slightly basic pH allowing the formation of biogas from the fermenting organic material by methanogenic microorganisms to occur under anaerobic conditions. Importantly, the pH of the fermenting material in the biogas reactor is kept at pH values of between 6.5 to 8.5, preferably between 7 to 8, more preferably around 7.5. This is of importance as such pH values allow the formation of methane to stably proceed since the methanogenic microorganisms are sensitive to acidic pH values. The acidic liquid fraction from the first reactor is thus added in a controlled manner to the second reactor to keep this biogas reactor at the indicated pH values. Importantly, to maintain the pH of the biogas reactor at the indicated range, the feeding of the second reactor is pH-controlled meaning the acidified liquid fraction is fed into the second reactor in such a manner that the pH of the contents of the second reactor does not fall below pH 6.5 or preferably 7. It is also possible to convey the filtered, acidified liquid fraction obtained from the filtering means into the second reactor.

A second consequence of the basic pH of the second reactor, next to the production of biogas, is that more than 85% of the phosphate precipitates, mainly as struvite. Also ammonium taranakite, potassium taranakite, brushite, and the like, can be formed. The "biogas reactor" thus also functions to obtain phosphorus-comprising precipitates.

Thus, in an advantageous embodiment of the present invention, the liquid fraction obtained by step b) or the filtered liquid fraction obtained from a filtering means is fed into the second reactor of step c) such that the pH of the contents of the second reactor does not become less than pH 6.5, preferably not less than pH 7, more preferably not less than pH 7.5.

After the acetogenic and methanogenic phases, and thus the production of biogas, the obtained liquid fraction can be further processed to obtain a fertilizer product that can be used in the agricultural sector. Using the method of the present invention also a potassium-enriched mineral concentrate can be obtained. Furthermore, the liquid fraction can be stripped to isolate ammonia therefrom or biologically treated using nitrification/denitrification processes to remove ammonia. Also, the liquid fraction can be concentrated by filtering, if desired, to remove organic particles followed by reverse-osmosis or by other means to obtain a mineral-rich concentrate.

The second reactor can comprise a fluidized bed reactor, such as the Crystalactor of DHV, or a Continuously-Stirred Tank Reactor (CSTR) including a settler.

The fermentation process according to the present invention can proceed under mesophilic (meaning herein that the fermentation process proceeds at a temperature of between 30 and 40°C) and/or thermophilic (meaning herein that the fermentation process proceeds at a temperature of between 50-60°C) conditions. More in particular, the fermentation in the first, acidifying reactor occurs mesophilically or preferably thermophilically, while the fermentation in the second reactor preferably proceeds mesophilically which ensures a more stable methane formation process. However, the fermentation in the second reactor may also proceed thermophilically. At thermophilic conditions, the contents are kept in the first reactor for 2-7 days, preferably 2-5 days, more preferably 2-3 days. When mesophylically operated, this period comprises 4-12 days, alternatively 7-10, or 6-8 days. For the second reactor, the retention times comprise from 2-10 days, preferably 2-7 days, more preferably 2-5 days for thermophilic conditions and 2-14 days, preferably 2-10, more preferably 2-7 or 3-6 days for mesophilic conditions. An advantage of the present invention is that the total retention time of the manure and/or biomass is shortened compared to conventional fermentation methods.

The liquid fraction obtained by step c) is added to a third reactor, which preferably comprises a fluidized bed reactor, which third reactor comprises liquid fraction having a pH of more than pH 8.5, preferably more than pH 9, to allow dissolved phosphates which may still be present to precipitate.

The third reactor is used in case the liquid fraction obtained from the second reactor still comprises sufficient amounts of fatty acids and/or dissolved phosphates to make it commercially or technically desirable to subject the material from the second reactor to the increased pH of the third reactor. At the increased pH levels, phosphates are allowed to mineralize into, *inter alia,* struvite.

Also, when the liquid fraction from the second and/or third reactor is circulated in the fermentation process, a potassium-enriched liquid fraction can be obtained which can be applied as fertilizer for crops requiring increased potassium levels.

In an advantageous embodiment, the formation of struvite crystals in the second and/or third reactor is facilitated by addition of seed crystals to any of these reactors and/or a solution comprising magnesium salts and/or calcium salts. Also, compounds or solutions can be added to the third reactor to further increase the pH of this reactor.

In yet another advantageous embodiment, to the first reactor used in step a) is added material obtained from a premixing container. This premixing container comprises a solid fraction of biomass and/or separated manure, preferably unfermented manure, mixed with liquid fraction obtained by step b) and/or the liquid fraction obtained from the washer/separator used to wash the acidified solid fraction and/or liquid fraction which recycles from the second and/or third reactor.

The main advantage of using a premixing container is that the pH of the solid fraction of the fresh, unfermented manure can be pre-acidified. This allows the acidifying reactions in the first reactor to proceed at lower pH values after the addition of unfermented material than would be the case when a solid fraction of fresh manure would not be premixed with acidified liquid fraction. Preferably, the material added to the first reactor obtained from the premixing container comprises a dry matter content of 15 to 20 percentage by weight. The dry matter content of this material is measured by the electricity consumption of the engine powering a mixing device present in the premixing container.

In an advantageous embodiment, is added to the premixing container, preferably in dependency of the pH of the contents of the premixing container, the following material: the liquid fraction obtained by step b), and/or the liquid fraction obtained from the washer/separator used to wash the acidified solid fraction, and/or liquid fraction obtained from the second reactor and/or liquid fraction obtained from the third reactor. This ensures sufficient control over the pH of the contents of the premixing container which allows controlling of the fermentation processes. Preferably, the premixing container comprises solid fraction of separated manure and/or biomass when the materials according to this embodiment are added to the premixing container.

Also, the feeding of the premixing container with the liquid fraction obtained by step b) is preferably pH regulated.

Disclosed is an installation for processing of manure and/or biomass according to the method of the present invention, comprising a first fermentation reactor, preferably an acidifying reactor suitable for allowing anaerobic fermentation to occur, having at least one inlet and at least one outlet, of which one outlet is connected via a suitable conduit to an inlet of a separator for separating acidified manure and/or biomass into a liquid and a solid fraction, the separator having a first outlet for transport of the solid fraction out of the separator and a second outlet for outflow of the liquid fraction, which second outlet is connected via a suitable conduit to an inlet of a second fermentation reactor, preferably a biogas reactor suitable for allowing anaerobic fermentation to proceed, for further fermenting of the liquid fraction at a pH between 6.5 and 8.5, which second reactor comprises at least one inlet and at least two outlets. Preferably, the rate of outflow through the outlet of the first reactor connected to the separator can be regulated by the pH of the contents of the first reactor, and wherein preferably the rate of inflow through the inlet of the second reactor connected to the separator for receiving liquid fraction can be regulated by the pH of the contents of the second reactor. Suitable means for allowing such pH controlled in- or outflow rates are known in the art and include a pH regulated flow indicator controller (PHFIC).

The second reactor comprises at least two outlets; one outlet for obtaining phosphorus-comprising crystals which have precipitated from the liquid fraction, such as struvite, and a second outlet for outflow of the liquid fraction.

The first reactor and the separator of the installation can be in fluidized connection between one outlet of the first reactor and the inlet of the separator via a suitable conduit. As the respective inlets and outlets can be closed, this allows regulation of flow from the first reactor to the separator.

The installation can comprise a third reactor for further processing of the liquid fraction obtainable from the second reactor. This reactor, preferably a fluidized bed reactor, has at least one inlet and at least two outlets connected to an outlet of the second reactor via a suitable conduit. The third reactor preferably has a lower capacity than the second reactor. The third reactor further comprises an outlet for obtaining precipitated phosphorus-comprising crystals, such as struvite.

The installation can further comprise a premixing container for premixing unfermented manure, preferably the solid fraction thereof, which premixing container comprises at least one outlet which is connected via a suitable conduit to an inlet of the first reactor, which premixing container comprises a first inlet connected via a suitable conduit with an outlet of the separator for receiving liquid fraction from the separator, which premixing container preferably comprises a second inlet connected via a suitable conduit with an outlet of a washer/separator to receive liquid fraction from the washer/separator, which washer/separator is intended to wash and again separate the solid fraction obtained from the separator, and/or which premixing container preferably comprises a third inlet connected via a suitable conduit with an outlet of the second and/or third reactor, wherein preferably the rate of inflow through the first inlet of the premixing container can be regulated by the pH of the contents of the premixing container.

An inlet of the first reactor can be equipped with a level-indicator controller (LIC), wherein this inlet is preferably connected to an outlet of the premixing container to allow controlled inflow in the first reactor of material from the premixing container.

The first and/or second and/or third reactor and/or premixing container can comprise means for mixing the contents thereof and/or a heat exchanger to allow the contents to be kept at a desired temperature.

In case the first reactor is operated as a plug-flow reactor, the mixing means only allow mixing of material having a similar retention time to keep fermenting material with different retention times separated. The concepts and use of plug-flow reactors and continuously-stirred reactors are well known in the art.

The separator for separating fermented manure into a liquid and a solid fraction can comprise a filtration or centrifugation means, preferably selected from a particle filter, a vibrating sieve, a curved screen, a screw press, a press auger, and a belt filter.

The filtrating device of the installation, preferably a microfiltrating device, is connected to an outlet of the separator for receiving liquid fraction therefrom and with an inlet of the second reactor, preferably the pH-regulable inlet.

The installation may further comprise a washer/separator intended to wash and again separate the solid fraction obtained from the separator, which washer/separator is connected via a suitable conduit with one inlet to an outlet of the separator for receiving solid fraction from the separator, and connected via a suitable conduit with one inlet to a water-source for receiving water and/or one inlet for the recirculation of liquid fraction from the second and/or third reactor, and/or connected via a suitable conduit with one outlet thereof to an inlet of the premixing container for supply of the washing water to the premixing container. The washer/separator further comprises an outlet for release of the solid fraction.

The installation can further comprise at least one buffering container, which container comprises at least one inlet and at least one outlet, wherein an inlet is connected to an outlet of the filtrating device and an outlet is connected to an inlet of the second reactor, and/or wherein one inlet is connected to one outlet of the separator for receiving liquid fraction and one outlet is connected to one inlet of the second reactor. Additionally or alternatively, a buffering container is comprised by the installation arranged such that one inlet of the buffering container is connected to one outlet of the second reactor, possibly also to an outlet of the optional third reactor, for receiving liquid fraction, and that one outlet is connected to an inlet of the premixing container and/or the washer/separator. Additionally or alternatively, a buffering container is comprised by the installation arranged such that an inlet thereof is connected to an outlet of the first reactor for receiving acidified manure and/or biomass, and an outlet thereof is connected to an inlet of the separator.

Additionally or alternatively, one buffering container is comprised by the installation and arranged such that an inlet thereof is connected to an outlet of the washer/separator and an outlet thereof is connected to one inlet of the premixing container. These buffering containers are present to allow fluctuations in the in- and outflow of the different units in the installation to be levelled and controlled.

The installation can be equipped with a flow-indicator controller (FIC) for measuring the outflow of liquid out of the installation. The installation comprises a closable outflow valve to allow outflow from the installation. Preferably, this outflow valve is placed between an inlet of the premixing container and an outlet of the second or third reactor. The FIC is then placed such that the outflow from this outflow valve can be measured to determine to total amount of liquid that leaves the installation.

The installation can be equipped with a master/slave to allow the outflow of liquid out of the installation to be compensated by the inflow of water. Preferably this master/slave is connected to one inlet of the washer/separator to directly use the inflow of water in to the fermentation system. However, the inlet of water can also be connected to other units of the fermentation installation.

### Description of the figures

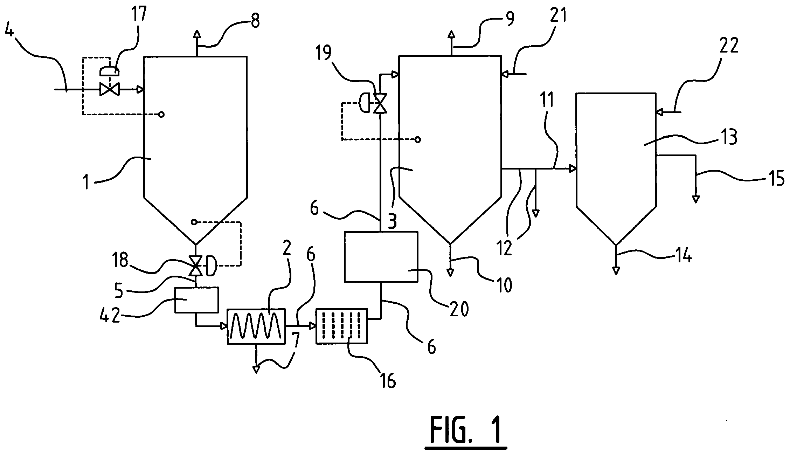

Figure 1 shows an installation comprising the acidifying reactor (1), the separator (2), the biogas reactor (3) and an optional third reactor (13). Conduit (4) is connected to an inlet of the first reactor (1). Conduit (5) is for transport of acidified organic matter to the separator (2) via buffering container (42). Conduit (6) is for transport of acidified liquid fraction to the biogas reactor (3) via optional filtrating device (16) and optional buffering container (20). Conduit (7) is for transport of solid fraction out of the separator (2). (8) Indicates gas produced during the fermentation process that occurs in the first reactor (1). (9) Indicates gas produced during the fermentation process that occurs in the second reactor (3). Conduit (10) is connected to an outlet of the second reactor (3) and serves to obtain precipitated phosphorus-comprising compounds. Conduit (11) is for transport of liquid fraction to the optional third reactor (13). Conduit (12) is connected to conduit (11) and is for transport of liquid fraction out of the second reactor (3). Conduit (14) is connected to an outlet of the third reactor (13) and serves to obtain precipitated phosphorus-comprising compounds from this reactor. Conduit (15) is for transport of liquid fraction out of the third reactor (13). Level-indicator controller (LIC) (17) is for regulated inflow into the first reactor (1). Both (18) and (19) are pH regulated flow indicator controllers (PHFIC). Inlets into the second (3) and third reactors (13) for addition of material other than the liquid fraction from the first or second reactor are indicated with (21) and (22), respectively.

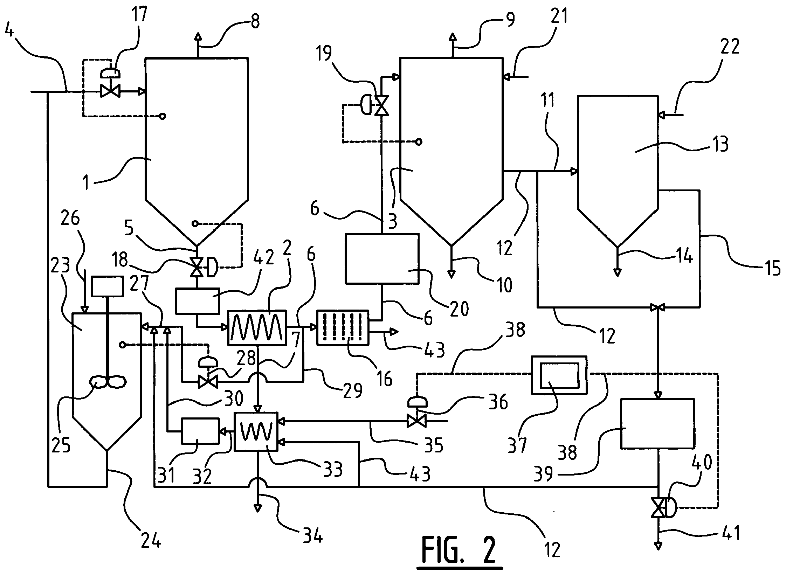

Figure 2 depicts an installation comprising the same components as shown in figure 1, with the following additions: premixing container (23) with stirring means (25), inlet (26) and pH regulated flow indicator controller (28), buffering container (31), washer/separator (33), water tap (36), master/slave (37), communication line (38) for allowing to compensate the amount of liquid leaving the installation via conduit (41) with the amount of water entering the installation via conduit (35) or vice versa, buffering container (39), flow-indicator controller (FIC) (40) for measuring the outflow of liquid out of the installation. Furthermore, the installation according to figure 2 comprises the following conduits: conduit (27) connected to an inlet of (23), conduits (24), (29), (30), (32), conduit (34) connected to an outlet of the washer/separator (33), conduit (35) for allowing water to enter the installation, and conduit (41) for allowing outflow of liquid out of the installation.

### Detailed description of Figure 1

With reference to figure 1, the method of the present invention is now described when manure and/or biomass is/are fermented and processed.

Biomass and/or manure is/are fed into the first reactor (1) wherein the acidifying reactions take place under anaerobic conditions. The organic material according to the invention is conveyed into the first reactor (1) through an inlet connected to conduit (4) which supply into reactor (1) is controlled by level-indicator controller (17). Fermentation in reactor (1) is allowed to proceed under anaerobic conditions until the organic matter inside this reactor reaches a pH of at least below 6. During these acidifying reactions gas, including CO₂ and H₂, escape from the organic matter (8). The first reactor comprises a pH regulated flow indicator controller (18) to ensure the organic matter which is conveyed to the separator (2) via conduit (5) has a pH of at least below 6. Preferably, the installation comprises a buffering container (42) which can be used to regulate the amount of material to be fed into the separator (2).

The separator (2) comprises an outlet (7) for transport of solid fraction out of the installation and an outlet for conveying acidified liquid fraction via conduit (6) to the second reactor (3), the biogas reactor. Optionally, conduit (6) is interrupted by a filtrating device (16) and/or a buffering container (20) which is/are arranged between the separator (2) and the second reactor (3).

The second reactor (3) comprises a pH regulated flow indicator controller (19) to allow pH-controlled supply of acidified liquid fraction via conduit (5). This allows feeding of acidified liquid fraction into the second reactor in a manner ensuring that the pH of the contents of the second reactor remains between pH 6.5 and pH 8.5. Liquid fraction which is in essence fully fermented is conveyed through an outlet into conduit (12), which conduit leads in an alternative embodiment to the third reactor (13) via conduit (11), or to buffering container (39), or premixing container (23), and/or washer/separator (33) and/or out of the installation via conduit (41). The components numbered with (39), (23), (33) and (41) are shown in Figure 2. Produced biogas (9) escapes from the liquid fraction. Outlet (10) allows to obtain precipitated phosphorus-comprising minerals, such as struvite. Inlet (21) allows addition of inoculum and/or other components as desired.

The optional third reactor (13) comprises outlet (14) for allowing to obtain precipitated phosphorus-comprising minerals, such as struvite. Inlet (22) can be used to add seed crystals and/or solutions comprising a magnesium salt and/or a calcium salt, if desired. Furthermore, the third reactor comprises an outlet leading into conduit (15), which conduit is connected to conduit (12) in figure 2. Inlet (22) allows addition of seed crystals and/or other components, as desired.

Filtration device (16), preferably a microfiltrating device, is optional and, when included in the installation, is connected to an outlet of the separator for receiving liquid fraction therefrom and connected to an inlet of the second reactor, preferably to an inlet which controlled by pH regulated flow indicator controller (19).

### Figure 2

With reference to figure 2 and the detailed description of figure 1, the method of the present invention is further described below when manure and/or biomass is/are fermented and processed.

The premixing container (23) is fed with the solid fraction of seperated unfermented manure and/or with biomass via inlet (26). According to figure 2, the premixing container comprises an inlet which is connected to conduit (27) which receives conduits (12), (29) and optionally also (32). Optionally, these conduits may each be directly connected to the premixing container. The contents of the premixing container can be stirred using stirrer (25).

When the first reactor has sufficient capacity for receiving manure and/or biomass for fermentation thereof, e.g. when the contents of reactor (1) are sufficiently acidified such that material can be conveyed to the separator, level-indicator controller (17) indicates that material from the premixing container can be fed into the first reactor.

The pH regulated flow indicator controller (28) functions such that the contents of the premixing container can be "pre-acidified" by addition of acidified liquid fraction derived from the separator means (2). This allows shortening of the retention time of organic matter in the first reactor.

The installation may further be equipped with buffering containers (31) and (39) which each allow upstream and/or downstream processes to proceed in a controlled manner. For example, buffering container (31) allows washing water to be retained from the washer/separator (33) to control inflow of liquid into the premixing container or to balance the addition of acidified liquid fraction supplied from separator (2). Buffering container (39) serves to retain processed liquid fraction obtained from reactors (3) and/or (13) and allows feeding the premixing container (23) with more alkaline liquid, if desired.

The solid fraction obtained from washer/separator (33) via (34) is sufficiently low on phosphorus and other nutrients to allow use thereof as fertilizer, optionally after composting thereof if desired.

Optional conduit (43) allows recycling of the liquid obtained from reactor (3), optionally also reactor (13), into the washer/separator (33) and feeding again into premixing container (23).

The installation according to the present invention is further equipped with a flow-indicator controller (FIC) (40) for measuring the outflow of liquid out of the installation. The installation comprises a closable outflow valve to allow outflow from the installation through conduit (41).

The installation according to figure 2 is further equipped with a master/slave (37) to allow balancing or compensating of inflow and outflow of liquids. Inflow of liquid, i.e. water, can be via a water tap (36) feeding into the washer/separator (33). Such a water tap can be alternatively placed to allow inflow of water in other components of the installation.

The invention is further illustrated by the following examples, which are by no means meant as a restriction of the invention, but merely intended to illustrate its practical use.

### EXAMPLE 1

### Materials and method

The experiment was performed in a stirred, heated airtight reactor with a content of 20 liters. The reactor was provided with a temperature control and stirring time programming. The tested manure came from pigs which were fed with fattening fodder.

The manure had the following characteristics. The dry matter content was 12% (percentage by weight), the pH of the manure was 8,2.

**Table 1. Characteristics of the tested pig manure.**

| parameter | Content (kg/m³) |
|---|---|
| P₂O₅ | 4,5 |
| Solids | 3,4 |
| K₂O | 6,0 |
| N total | 9,6 |

### Design of the tests and results

The reactor was filled with the characterized manure and acid inoculum and was kept airtight. An overpressure valve was installed for release of the formed gasses. The reactor was kept at 55 °C and the stirring program was started.

During the test the pH was measured and after 5 days the pH stabilized at 3.9. A well-mixed sample was obtained and analyzed. The manure was separated into a solid and a liquid fraction using a Millipore® sieve of 0,45 micron.

The following results were obtained, based on the ratio of calculated mass fractions.

**Table 2. Analysis of components present in the liquid fraction of acidified pig manure.**

| Parameter | Amount in liquid fraction (kg/m³) | Weight percentage |
|---|---|---|
| P₂O₅ | 3, 9 | 88% |
| K₂O | 5,4 | 91% |
| Nₜₒₜₐₗ | n.d. | |

The test revealed that approximately 90% of the phosphate is present in the liquid fraction of treated pig manure. It is also possible to wash the solid fraction with water, and again separate the washed solid fraction into a solid and liquid fraction in which case the amount of phosphate in the liquid fraction would be even higher.

### EXAMPLE 2

In a second experiment, a mixture of manure and biomass was placed under anaerobic conditions to allow fermentation and acidification thereof to measure the distribution of phosphorus in solid and liquid fractions after acidification of the manure/biomass mixture has taken place.

### Starter material

A mixture of approximately 2.8 kg comprising of 1 part by weight inoculum from a fermentation reactor wherein a carbohydrate-rich stream of organic material was co-fermented under acidifying conditions, was mixed with 2 parts by weight of starch and 8 parts by weight of fresh pig manure, providing a starter material of 1:2:8 parts by fresh weight.

The starter material was characterized by the parameters according to table 3.

**Table 3: Characteristics of substrates and inoculum.**

| | Dry matter (DM) (g/kg) | Organic matter (OM) (g/kg) | Percentage OM of DM (%) |
|---|---|---|---|
| Manure | 99.1 | 74.6 | 75.2 |
| Starch | 815 | 812 | 100 |
| Inoculum | 59.8 | 41.8 | 70.0 |

The mixture was approximately equally divided over two 2 L sealable flasks, to which approximately 1 kg of the mixture was added. The flasks were incubated under mesophilic conditions, under continuous shaking. Prior to the incubation, the atmospheric gas phase was replaced with nitrogen gas.

To measure the relevant parameters of the solid and liquid fractions, part of the mixture was centrifuged for 15 minutes at 2500 rpm, separated and analyzed for dry matter, organic matter, pH, volatile fatty acids (in particular fatty acids having 2-5 carbon atoms), phosphates and phosphorus. Also the non-separated material was analysed. The pH was measured in time throughout the experiment. After the acidifying test, each of the duplicates were separated and analyzed in the same manner to allow proper comparison of the results. Furthermore, the mixtures were analysed.

### Analyses

The dry matter and organic matter was measured using standard methods. Samples were dried at 105ºC and incinerated at 550ºC.

Dissolved volatile fatty acid content was measured using gas chromatography. Sample preparation consisted of a 10 minute centrifugation step at 10.000 rpm and dilution of the supernatant with 3% formic acid. Using this method, short-chained fatty acids, such as acetic acid, propionic acid, butyric acid and valeric acid were measured.

Phosphate-P was measured after 10 minute centrifugation at 10.000 rpm, followed by dilution in demineralized water to ensure the parameters were within measuring range and spectro-photometrically determined with a Skalar autoanalyser.

Total-P was photometrically measured using standard methods, i.e. WI 4.25-104 and WI 4.25-106.

### Results

The results show that the pH of the contents of the flasks rapidly drops from pH 8 to pH 4.4 within 8 days after the start of the test.

**Table 4: progression of the pH during the incubation of the mixtures of Example 2**

| Time (days) | Flask 1 | Flask 2 |
|---|---|---|
| t=0.0 | 7.96 | 7.96 |
| t=0.9 | 8.04 | 8.03 |
| t=2.3 | 6.88 | 6.97 |
| t=3.0 | 5.83 | 5.85 |
| t=4.1 | 5.42 | 5.44 |
| t=6.0 | 4.73 | 4.70 |
| t=7.2 | 4.46 | 4.44 |
| t=8.0 | 4.45 | 4.40 |

What is demonstrated is that the pH of the contents of the flasks rapidly acidifies and appears to reach a plateau at around pH 4.4 after 7 or 8 days.

Also, the contents of the flask at t=0 were analyzed and compared to the contents of the flasks after the incubation period at t=8 days.

**Table 5. Parameters of the mixture and different fractions derived therefrom at t=0. OM = Organic Matter, DM = Dry Matter. <dl = below detection limit, - = not measured.**

| | Units | mixture | Solid fraction | Liquid fraction |
|---|---|---|---|---|
| P-total | g P/kg | 1.95 | 2.99 | 0.45 |
| P-PO₄³⁻ | mg P/l | 305 | - | 294 |
| DM | g/kg | 225 | 389 | 33.7 |
| OM | g/kg | 205 | 363 | 21.8 |
| OM | % of DS | 91.2 | 93.3 | 64.7 |
| Acetic acid | mg/l | 358 | - | - |
| Priopionic acid | mg/l | 54 | - | - |
| Butyric acid | mg/l | <dl | - | - |

**Table 6. Parameters of the mixture and different fractions derived therefrom at t=8 days. The units of the measured parameters are the same as shown in table 5. OM = Organic Matter, DM = Dry Matter, - = not measured.**

| | Mixture | | Solid fraction | | Liquid fraction | |
|---|---|---|---|---|---|---|
| | F1. 1 | F1. 2 | F1. 1 | F1. 2 | F1. 1 | F1. 2 |
| P-total | 1. 97 | 1.93 | 1.72 | 1.69 | 2.07 | 2.07 |
| P-PO₄³⁻ | 1878 | 1977 | - | - | 2050 | 1853 |
| DM | 202 | 202 | 350 | 351 | 113 | 115 |
| OM | 181 | 181 | 330 | 330 | 92.0 | 93.3 |
| OM | 89.6 | 89.7 | 94.2 | 94.1 | 81.2 | 81.3 |
| Acetic acid | 18491 | 19186 | - | - | - | - |
| Priopionic acid | 2683 | 2852 | - | - | - | - |
| Butyric acid | 819 | 928 | - | - | - | - |

The results clearly show that the P-total had shifted dramatically to the liquid fraction after acidification of the contents of the flasks. Interestingly, after acidification the amount of soluble phosphates nearly equals the amount of total phosphorus, both as measured in the mixture and in the liquid fraction (on average 1952 mg P-P043- 1-1 vs. 2.07 g P-total kg-1 as measured in the liquid fraction). Thus, it can be concluded that after acidification nearly all phosphorus is present in the form of dissolved phosphates. The distribution of dissolved phosphate over the solid and the liquid fraction is directly related to the volume ratio between the solid and the liquid fraction. This, in turn, is dependent on the separation means and the DM content of the biomass. In the example of the experiment we used a mixture with a relatively high DM content (22.5%), and consequently a relatively high amount of phosphorus remained in the solid fraction. However, when using biomass with a DM content of 10% before separation, and after separation a solid fraction with 25-30% DM is obtained, the total phosphate that remains the solid fraction will be <10%. Furthermore, the amount of phosphorus in the solid fraction can be further reduced by means of washing this fraction with water.

### Example 3

In order to gain more insight in the precipitation of phosphate as a function of the pH, an experiment has been executed with very old pig manure, with a DM content of 134 g/kg. This manure was already fermented during storage on the farm and practically all phosphate was present in precipitated form such as struvite etc. In the laboratory this manure was separated into a solid fraction and a liquid fraction by 20 minutes centrifugation at 2395 rcf. The resulting solid fraction had a DM content of 247 g/kg and a phosphate content of 5,6 g P/kg. This solid fraction was diluted with 2M acetic acid and water to obtain solid fractions with different pH values (pH 7.0, 6.5 and 6.0), with a dry matter content of 15%. In experiment 1, no acetic acid was added, but the solid fraction was diluted with water such that a DM content of 15% was obtained. The mixtures were analysed for phosphate and acid content directly after reaching the desired pH, results are presented in Table 7.

**Table 7. Phosphate precipitation in the solid fraction of old fermented manure as a function of the pH.**

| | pH | P-total (g P/kg) | P-PO4 (mg P/l) | Acetic acid (mg/l) | Propionic acid (mg/l) | Butyric acid (mg/l) |
|---|---|---|---|---|---|---|
| Exp.1 | 8.7 | 3.4 | 192 | 159 | 7 | 184 |
| Exp.2 | 7.0 | 3.4 | 236 | 8966 | 29 | 193 |
| Exp.3 | 6.5 | 3.4 | 339 | 15242 | 48 | 208 |
| Exp.4 | 6.0 | 3.4 | 836 | 21889 | 69 | 247 |

### Conclusions

From the table above it becomes clear that the solubility of phosphate at pH 6.5 and higher is very low, lower than 10% of the total amount of phosphorus. In the test at pH 6, the solubility of phosphate is approximately 25% of the total amount of phosphorus. It can be concluded that in experiment 2 at a pH higher than 6.5 practically all phosphate precipitates. Therefore, the production of biogas, which preferably occurs at pH 7.5, does not interfere with the formation of struvite etc.

## Claims

1. Method for dephosphorizing manure, human excreta or sludges derived from municipal wastewater treatment, comprising the steps of:
a) fermenting at anaerobic conditions the manure, human excreta or sludges derived from municipal wastewater treatment, preferably a solid fraction thereof, with addition of phosphate-enriched liquid fraction obtained by step b) or energy-rich fermentable biomass, in a first reactor to obtain acidified manure, human excreta, or sludges derived from municipal wastewater treatment having a pH of below 6, preferably below pH 5.5, preferably below pH 5, which allows the provision of a phosphate-poor solid fraction and a phosphate-enriched liquid fraction,
b) separating the phosphate-poor solid fraction and the phosphate-enriched liquid fraction, preferably without an intermediate processing step,
c) adding the phosphate-enriched liquid fraction to a second reactor, which second reactor comprises fermenting liquid fraction having a pH of between 6.5 to 8.5, preferably 7.0 to 8.0, and fermenting the thus obtained mixture in the second reactor, wherein the fermenting material is kept at pH values of between 6.5 to 8.5;
d) adding the liquid fraction obtained by step c) to a third reactor, which preferably comprises a fluidized bed reactor, which third reactor comprises a liquid fraction having a pH of more than pH 8.5 to allow dissolved phosphates to precipitate, and
e) recovering/obtaining precipitated phosphorus-comprising crystals, such as struvite,
wherein the phosphate-poor solid fraction obtained by step b) is washed with water and separated again in a solid and liquid fraction.

2. Method according to claim 1, wherein the manure is from farm animals, in particular pigs or cows.

3. Method according to claim 1 or 2, wherein the phosphate-enriched liquid fraction obtained by step b) is filtered prior to addition thereof to the second reactor used in step c), which filtering preferably comprises microfiltration, preferably wherein the second reactor comprises a fluidized bed reactor or a continuous-stirred tank reactor including a settler.

4. Method according to any of the claims 1-3, wherein formation of phosphate-comprising crystals, preferably struvite crystals, in the second or third reactor is facilitated by addition thereto of seed crystals or a solution comprising a magnesium salt or a solution comprising a calcium salt.

## Patentansprüche

1. Verfahren zur Entphosphorung von Gülle, menschlichen Exkrementen oder Schlämmen, die aus einer städtischen Abwasserbehandlung resultieren, mit den Schritten:
a) Fermentieren der Gülle, der menschlichen Exkremente oder der Schlämme, die aus der städtischen Abwasserbehandlung resultieren, vorzugsweise ihrer festen Fraktion, unter anaeroben Bedingungen unter Zusatz einer phosphatsreichen flüssigen Fraktion, die im Schritt b) erhalten wird, oder einer energiereichen fermentierbaren Biomasse in einem ersten Reaktor, um versauerte Gülle, menschliche Exkremente oder Schlämme, die aus einer städtischen Abwasserbehandlung resultieren, mit einem pH von unter 6, vorzugsweise unter pH 5,5, vorzugsweise unter pH 5 zu erhalten, was das Schaffen einer phosphatarmen festen Fraktion und einer phosphatreichen flüssigen Fraktion erlaubt,
b) Trennen der phosphatarmen festen Fraktion und der phosphatreichen flüssigen Fraktion, vorzugsweise ohne einen Behandlungs-Zwischenschritt,
c) Zufügen der phosphatreichen flüssigen Fraktion in einen zweiten Reaktor, wobei der zweite Reaktor eine flüssige Fermentationsfraktion mit einem pH von zwischen 6,5 bis 8,5, vorzugsweise 7,0 bis 8,0 aufweist, und Fermentieren der so erhaltenen Mischung in dem zweiten Reaktor, wobei das Fermentationsmaterial bei pH-Werten von zwischen 6,5 bis 8,5 gehalten wird,
d) Zufügen der flüssigen Fraktion, die im Schritt c) erhalten wird, in einen dritten Reaktor, der vorzugsweise einen Flussbett-Reaktor aufweist, wobei der dritte Reaktor eine flüssige Fraktion mit einem pH von mehr als pH 8,5 aufweist, um zu ermöglichen, dass gelöste Phosphate ausfällen, und
e) Wiederherstellung/Erhalten ausgefällter phosphathaltiger Kristalle wie Struvit, wobei die phosphatarme feste Fraktion, die im Schritt b) erhalten wird, mit Wasser gewaschen und erneut in eine feste und eine flüssige Fraktion getrennt wird.

2. Verfahren nach Anspruch 1, wobei die Gülle von Nutztieren, insbesondere von Schweinen oder Rindern stammt.

3. Verfahren nach Anspruch 1 oder 2, wobei die phosphatreiche flüssige Fraktion, die im Schritt b) erhalten wird, vor ihrem Zusatz zu dem zweiten Reaktor, der im Schritt c) verwendet wird, gefiltert wird, wobei die Filterung vorzugsweise eine Mikrofiltration aufweist, wobei vorzugsweise der zweite Reaktor einen Flussbett-Reaktor oder einen Tankreaktor mit kontinuierlichem Rühren einschließlich eines Abscheiders aufweist.

4. Verfahren nach einem der Ansprüche 1-3, wobei die Bildung von phosphathaltigen Kristallen, insbesondere von Struvitkristallen in dem zweiten oder dem dritten Reaktor durch Zusatz von Keimkristallen oder einer Lösung, die ein Magnesiumsalz aufweist, oder einer Lösung, die ein Kalziumsalz aufweist, erleichtert wird.

## Revendications

1. Procédé pour déphosphorer du fumier, des excréments humains ou des boues dérivées d'un traitement des eaux usées municipales, comprenant les étapes consistant à :
a) fermenter dans des conditions anaérobie le fumier, les excréments humains ou les boues dérivées d'un traitement des eaux usées municipales, de préférence une fraction solide de ceux-ci, avec l'addition d'une fraction liquide enrichie en phosphate obtenue par l'étape b) ou d'une biomasse fermentable riche en énergie, dans un premier réacteur afin d'obtenir du fumier, des excréments humains, ou des boues dérivées d'un traitement des eaux usées municipales acidifiés ayant un pH inférieur à 6, de préférence un pH inférieur à 5,5, de préférence un pH inférieur à 5, qui permet d'obtenir une fraction solide pauvre en phosphate et une fraction liquide enrichie en phosphate,
b) séparer la fraction solide pauvre en phosphate et la fraction liquide enrichie en phosphate, de préférence sans étape de traitement intermédiaire,
c) ajouter la fraction liquide enrichie en phosphate à un deuxième réacteur, lequel deuxième réacteur comprend la fermentation de la fraction liquide ayant un pH compris entre 6,5 et 8,5, de préférence 7,0 à 8,0, et fermenter le mélange ainsi obtenu dans le deuxième réacteur, où le matériel de fermentation est maintenu à des valeurs de pH comprises entre 6,5 et 8,5 ;
d) ajouter la fraction liquide obtenue par l'étape (c) à un troisième réacteur, qui comprend de préférence un réacteur à lit fluidisé, lequel troisième réacteur comprend une fraction liquide ayant un pH supérieur à un pH 8,5 afin de permettre aux phosphates dissous de précipiter, et
e) récupérer/obtenir des cristaux comprenant du phosphore précipités, comme de la struvite, où la fraction solide pauvre en phosphate obtenue par l'étape b) est lavée avec de l'eau et à nouveau séparée en une fraction solide et liquide.

2. Procédé selon la revendication 1, dans lequel le fumier provient d'animaux d'élevage, en particulier de porcs ou de vaches.

3. Procédé selon la revendication 1 ou 2, dans lequel la fraction liquide enrichie en phosphate obtenue par l'étape b) est filtrée avant l'addition de celle-ci au deuxième réacteur utilisé à l'étape c), laquelle filtration comprend de préférence une microfiltration, de préférence où le deuxième réacteur comprend un réacteur à lit fluidisé ou un réacteur à cuve agitée en continu comprenant un décanteur.

4. Procédé selon l'une quelconque des revendications 1 à 3, dans lequel la formation de cristaux comprenant du phosphate, de préférence des cristaux de struvite, dans le deuxième ou troisième réacteur, est facilitée par l'addition à celui-ci de germes cristallins ou d'une solution comprenant un sel de magnésium ou d'une solution comprenant un sel de calcium.
